(19) Europäisches Patentamt
European Patent Office
Office européen des brevets

(11) Veröffentlichungsnummer: **0 363 643 A1**

# (12) EUROPÄISCHE PATENTANMELDUNG

(21) Anmeldenummer: **89116497.2**

(22) Anmeldetag: **07.09.89**

(51) Int. Cl.5: **C07D 471/04 , A01N 43/90 , //(C07D471/04,221:00,221:00)**

(30) Priorität: **20.09.88 DE 3831914**

(43) Veröffentlichungstag der Anmeldung: **18.04.90 Patentblatt 90/16**

(84) Benannte Vertragsstaaten: **BE CH DE FR GB IT LI NL**

(71) Anmelder: **BAYER AG**
**D-5090 Leverkusen 1 Bayerwerk(DE)**

(72) Erfinder: **Heinemann, Ulrich, Dr.**
**Feldstrasse 18**
**D-5653 Leichlingen 1(DE)**
Erfinder: **Brandes, Wilhelm, Dr.**
**Eichendorffstrasse 3**
**D-5653 Leichlingen(DE)**
Erfinder: **Dutzmann, Stefan, Dr.**
**Leinenweberweg 33**
**D-4000 Düsseldorf 13(DE)**

## (54) Schädlingsbekämpfungsmittel auf Basis von teilweise neuen 2-Aryl-substituierten 1,10-Phenanthrolinen und deren Herstellung.

(57) Verwendung von 2-Aryl-substituierten 1,10-Phenanthrolinen der Formel

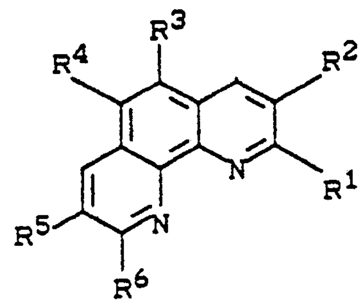

worin $R^1$ bis $R^6$ die in der Beschreibung angegebene Bedeutung haben zur Bekämpfung von Schädlingen sowie neue 2-Aryl-substituierte 1,10-Phenanthroline.

Die 2-Aryl-substituierten 1,10-Phenanthroline können hergestellt werden z. B. durch Cyclisierung von geeigneten α-Aminochinolinen mit geeigneten α,β-ungesättigten Aldehyden gegebenenfalls in Gegenwart geeigneter Verdünnungsmittel sowie gegebenenfalls in Gegenwart einer geeigneten starken Säure oder eines geeigneten Oxidationsmittels oder unter Wasserabscheidung gegebenenfalls in Gegenwart eines geeigneten Katalysators.

EP 0 363 643 A1

# Schädlingsbekämpfungsmittel auf Basis von teilweise neuen 2-Aryl-substituierten 1,10-Phenanthrolinen und deren Herstellung

Die vorliegende Erfindung betrifft die Verwendung von teilweise bekannten 2-Aryl-substituierten 1,10-Phenanthrolinen zur Bekämpfung von Schädlingen sowie neue 2-Aryl-substituierte 1,10-Phenanthroline und mehrere Verfahren zu ihrer Herstellung.

Es ist bereits bekannt, daß bestimmte Tetrahydrophthalimide, wie beispielsweise cis-N-[-(Trichlormethyl)thio]-4-cyclohexen-1,2-dicarboimid, fungizide Eigenschaften aufweisen (vgl. z.B. Science, (Washington) 115, 84 (1952); US 2 553 770).

Weiterhin ist bereits bekannt, daß bestimmte Perhalogenalkylmercapto-sulfonamide und -sulfamide, wie beispielsweise, N,N-Dimethyl-N′-phenyl-N′-(fluordichlormethylthio)-sulfamid, fungizide Eigenschaften aufweisen (vgl. DAS 1 193 498).

Die Wirkung dieser Verbindungen ist jedoch insbesondere bei niedrigen Aufwandmengen und -konzentrationen nicht immer zufriedenstellend.

Weiterhin werden 2-Aryl-substituierte 1,10-Phenanthroline beschrieben (vgl. J. Am. Chem. Soc., 110, 1467-72, 1988; Tetrahedron Lett., 24, 5091-4, 1983; Tetrahedron Lett., 23, 5291-4, 1982; CS-PS 150747; Tetrahedron Lett., 43, 895-904, 1987; Tetrahedron, 43, 333-44, 1987; Tetrahedron Lett., 27, 2257-60, 1986; Tetrahedron Lett., 27, 865-8, 1986; J. Am. Chem. Soc., 106, 3043-5, 1984; Tetrahedron Lett., 24, 5095-8, 1983; J. Chem. Soc., Chem. Commun., 1376-8, 1986; J. Phys. Chem., 60, 1546-8, 1956, deren Wirksamkeit im Pflanzenschutz ist jedoch nicht bekannt.

Es wurde gefunden, daß die teilweise bekannten 2-Aryl-substituierten 1,10-Phenanthroline der allgemeinen Formel (I),

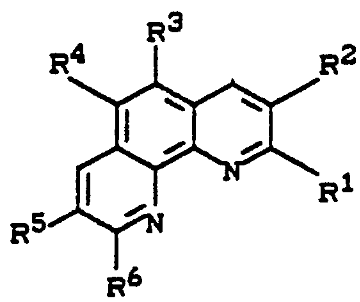

(I)

in welcher

$R^1$ für Wasserstoff, Alkyl oder unsubstituiertes oder substituiertes Phenyl steht,

$R^2$ und $R^5$ gleich oder verschieden sind und für Wasserstoff oder Alkyl stehen,

$R^3$ und $R^4$ gleich oder verschieden sind und für Wasserstoff, Alkyl, Halogenalkyl, Halogen, Hydroxy, Alkoxy, Mercapto, Alkylthio, Alkylsulfinyl, Alkylsulfonyl, Nitro, unsubstituiertes oder substituiertes Aryl, Amino, Alkylamino, unsubstiutiertes oder substituiertes Arylamino, Aralkylamino, Dialkylamino, Diaralkylamino oder für die Gruppierungen $-NH-CO-R^7$, $-NH-CY-X-R^8$, $-NH-SO_2-R^9$, $-Y^1-CO-R^{10}$, $-Y^2-CO-X^1-R^{11}$, $-Y^3-CS-X^2-R^{12}$ oder $-CO-R^{13}$ stehen,

worin

$R^7$, $R^8$, $R^9$, $R^{10}$, $R^{11}$, $R^{12}$ und $R^{13}$ gleich oder verschieden sind und für Alkyl oder unsubstituiertes oder substituiertes Phenyl stehen,

Y, $Y^1$, $Y^2$ und $Y^3$ gleich oder verschieden sind und für Sauerstoff oder Schwefel stehen und

X, $X^1$ und $X^2$ gleich oder verschieden sind und für Sauerstoff, Schwefel oder für die Gruppe $N-R^{14}$ stehen, worin $R^{14}$ für Wasserstoff oder Alkyl steht,

und

$R^6$ für unsubstituiertes oder substituiertes Phenyl steht,

sowie deren Säureadditions-Salze und Metallsalz-Komplexe überraschenderweise ausgezeichnete biologische Eigenschaften besitzen.

So zeigen die erfindungsgemäß zu verwendenden 2-Aryl-substituierten 1,10-Phenanthroline der Formel (I) sowie deren Säureadditions-Salze und Metallsalz-Komplexe eine bessere Wirkung gegen Schädlinge, insbesondere Pilze, als das aus dem Stand der Technik bekannte cis-N-[(Trichlormethyl)thio]-4-cyclohexen-1,2-dicarboimid und das N,N-Dimethyl-N′-phenyl-N′-(fluordichlormethylthio)sulfamid.

Die erfindungsgemäß zu verwendenden 2-Aryl-substituierten 1,10-Phenanthroline sind durch die Formel (I) allgemein definiert.

Alkyl steht im weiteren auch in alkylhaltigen Resten wie Alkylsulfinyl, Alkylamino, Aralkylamino, Dialkylamino, Diaralkylamino, Alkylsulfonyl, Alkoxy, Alkylthio und Halogenalkyl, falls nicht anders definiert, für geradkettiges oder verzweigtes Alkyl mit 1 bis 4 vorzugsweise 1 oder 2 Kohlenstoffatomen. Beispielhaft seien genannt: Methyl, Ethyl, n- und i-Propyl, n-, i-, s- und t-Butyl, Methoxy, Ethoxy, n- und i-Propyloxy, n-, i- und t-Butyloxy, Methylthio, Ethylthio, n- und i-Propylthio, n-, i- und t-Butylthio und Trifluormethyl.

Die Phenylreste der allgemeinen Formel können gegebenenfalls einen oder mehrere, vorzugsweise 1 bis 5, insbesondere 1 bis 3 gleiche oder verschiedene Substituenten tragen. Als Substituenten seien beispielhaft und vorzugsweise aufgeführt: Alkyl, Alkoxy, Halogenalkyl, insbesondere Trifluormethyl, Halogen, insbesondere Fluor, Chlor und Brom, Nitro, Hydroxy, sowie Alkinyloxy vorzugsweise 2-Propinyloxy.

Bevorzugt werden diejenigen Verbindungen der Formel (I) zur Schädlingsbekämpfung verwendet, in welcher
$R^1$ für Wasserstoff, geradkettiges oder verzweigtes Alkyl mit 1 bis 4 Kohlenstoffatomen, unsubstituiertes oder einfach oder mehrfach, gleich oder verschieden durch Halogen, Alkyl, Alkoxy oder Halogenalkyl mit jeweils bis zu 4 Kohlenstoffatomen substituiertes Phenyl steht,
$R^2$ und $R^5$ gleich oder verschieden sind und für Wasserstoff oder geradkettiges oder verzweigtes Alkyl mit 1 bis 4 Kohlenstoffatomen stehen,
$R^3$ und $R^4$ gleich oder verschieden sind und für Wasserstoff, für geradkettiges oder verzweigtes Alkyl mit 1 bis 4 Kohlenstoffatomen, Halogenalkyl mit 1 oder 2 Kohlenstoffatomen und 1 bis 5 gleichen oder verschiedenen Halogenatomen, Halogen, Hydroxy, Alkoxy oder Alkylthio mit jeweils 1 bis 4 Kohlenstoffatomen, Mercapto, Alkylsulfinyl oder Alkylsulfonyl mit jeweils 1 bis 4 Kohlenstoffatomen, Nitro, unsubstituiertes oder einfach oder zweifach, gleich oder verschieden durch Halogen, Alkyl oder Halogenalkyl mit jeweils 1 bis 3 Kohlenstoffatomen und 1 bis 5 gleichen oder verschiedenen Halogenatomen substituiertes Phenyl, ferner für Amino, für jeweils geradkettiges oder verzweigtes Alkylamino oder Dialkylamino mit jeweils 1 bis 4 Kohlenstoffatomen je Alkylteil, unsubstituiertes oder einfach oder zweifach, gleich oder verschieden durch Halogen, Alkyl oder Halogenalkyl mit jeweils 1 bis 3 Kohlenstoffatomen und gegebenenfalls 1 bis 5 gleichen oder verschiedenen Halogenatomen substituiertes Phenylamino, für jeweils unsubstituiertes oder im Phenylteil einfach bis mehrfach, gleich oder verschieden durch Halogen substituiertes Phenylalkylamino oder Diphenylalkylamino mit jeweils 1 oder 2 Kohlenstoffatomen im Alkylteil oder für die Gruppierungen -NH-CO-$R^7$, -NH-CY-X-$R^8$, -NH-$SO_2$-$R^9$, $Y^1$-CO-$R^{10}$, -$Y^2$-CO-$X^1$-$R^{11}$, -$Y^3$-CS-$X^2$-$R^{12}$ oder -CO-$R^{13}$ stehen, worin
$R^7$, $R^8$, $R^9$, $R^{10}$, $R^{11}$, $R^{12}$ und $R^{13}$ gleich oder verschieden sind und für geradkettiges oder verzweigtes Alkyl mit 1 bis 4 Kohlenstoffatomen oder für unsubstituiertes oder einfach bis dreifach, gleich oder verschieden durch Halogen, Nitro oder Alkyl mit 1 bis 4 Kohlenstoffatomen substituiertes Phenyl stehen,
Y, $Y^1$, $Y^2$ und $Y^3$ gleich oder verschieden sind und für Sauerstoff oder Schwefel stehen und
X, $X^1$ und $X^2$ gleich oder verschieden sind und für Sauerstoff, Schwefel oder für die Gruppe N-$R^{14}$ stehen, worin $R^{14}$ für Wasserstoff oder geradkettiges oder verzweigtes Alkyl mit 1 bis 4 Kohlenstoffatomen steht, wobei
$R^3$ und $R^4$ insbesondere gleich oder verschieden sind und für Wasserstoff, jeweils geradkettiges oder verzweigtes Alkyl, Alkoxy oder Alkylthio mit 1 bis 4 Kohlenstoffatomen oder für Fluor, Chlor oder Brom stehen und
$R^6$ für unsubstituiertes oder einfach oder mehrfach, gleich oder verschieden durch Halogen, Hydroxy, Alkyl, Alkoxy oder Halogenalkyl mit jeweils bis zu 4 Kohlenstoffatomen und gegebenenfalls 1 bis 5 gleichen oder verschiedenen Halogenatomen oder Alkinyloxy mit 2 bis 4 Kohlenstoffatomen substituiertes Phenyl steht.

Besonders bevorzugt werden diejenigen Verbindungen der Formel (I) verwendet, in welchen
$R^1$ für Wasserstoff, Methyl, Ethyl, n- und i-Propyl, n-, i-, s- und t-Butyl, unsubstituiertes oder einfach bis dreifach, gleich oder verschieden durch Fluor, Chlor, Brom, Methyl, Ethyl, Methoxy, Ethoxy oder Trifluormethyl substituiertes Phenyl steht,
$R^2$ and $R^5$ gleich oder verschieden sind und für Wasserstoff, Methyl, Ethyl, n- oder i-Propyl, n-, i-, s-und t-Butyl stehen,
$R^3$ und $R^4$ gleich oder verschieden sind und für Wasserstoff, Methyl, Ethyl, n- und i-Propyl, Fluor, Chlor, Brom, Hydroxy, Methoxy, Ethoxy, n-Propoxy, i-Propoxy, Mercapto, Methylthio, Ethylthio, n-Butylsulfinyl, Methylsulfonyl, Nitro, unsubstituiertes oder einfach oder zweifach, gleich oder verschieden durch Fluor, Chlor, Brom, Methyl, Ethyl, i-Propyl oder Trifluormethyl substituiertes Phenyl, ferner für Amino, Methylamino, Ethylamino, 2,6-Dichlorbenzylamino, Dimethylamino, Diethylamino, unsubstituiertes oder einfach oder zweifach, gleich oder verschieden durch Fluor, Chlor, Brom, Methyl, Ethyl, i-Propyl oder Trifluormethyl substituiertes Phenylamino oder für die Gruppierungen -NH-CO-$R^7$, -NH-CY-X-$R^8$, -NH-$SO_2R^9$, -$Y^1$-CO-$R^{10}$, -$Y^2$-CO-$X^1$-$R^{11}$, -$Y^3$-CS-$X^2$-$R^{12}$ oder -CO-$R^{13}$ stehen,

worin

$R^7$, $R^8$, $R^9$, $R^{10}$, $R^{11}$, $R^{12}$ und $R^{13}$ gleich oder verschieden sind und für Methyl, Ethyl, n- und i-Propyl, n-, i-, s- und t-Butyl oder für gegebenenfalls einfach oder zweifach, gleich oder verschieden durch Fluor, Chlor, Brom, Nitro, Methyl, Ethyl substituiertes Phenyl stehen,

Y, $Y^1$, $Y^2$ und $Y^3$ gleich oder verschieden sind und für Sauerstoff oder Schwefel stehen und

X, $X^1$ und $X^2$ gleich oder verschieden sind und für Sauerstoff, Schwefel oder für die Gruppe N-$R^{14}$ stehen, worin

$R^{14}$ für Wasserstoff oder Methyl steht, wobei

$R^3$ und $R^4$ insbesondere gleich oder verschieden sind und für Wasserstoff, Methyl, Ethyl, Methoxy, Ethoxy, Methylthio, Ethylthio, Fluor, Chlor oder Brom stehen und

$R^6$ für unsubstituiertes oder einfach bis dreifach, gleich oder verschieden durch Fluor, Chlor, Brom, Hydroxy, 2-Propinyloxy, Methyl, Ethyl, Methoxy, Ethoxy oder Trifluormethyl substituiertes Phenyl steht.

Bevorzugt erfindungsgemäß zu verwendende Verbindungen sind auch Additionsprodukte aus Säuren und denjenigen 2-Aryl-substituierten 1,10-Phenanthrolinen der Formel (I) in denen $R^1$, $R^2$, $R^3$, $R^4$, $R^5$ und $R^6$ diejenigen Bedeutungen haben, die im Zusammenhang mit der Beschreibung der erfindungsgemäß zu verwendenden Stoffe bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Malonsäure, Glutarsäure, Oxalsäure, Maleinsäure, Bernsteinsäure, Fumarsäure, Weinsäure, Zitronensäure, Salicylsäure, Sorbinsäure und Milchsäure, Ölsäure, Palmitinsäure, Stearinsäure, unsubstituierte oder einfach bis mehrfach durch Nitro oder Halogen substituierte Benzoesäure, Gluconsäure, Ascorbinsäure, Äpfelsäure, Sulfamidsäure, Sulfonsäuren, wie z.B. Benzol-1,3-disulfonsäure, Benzolsulfonsäure, Perfluorbutansulfonsäure, p-Toluolsulfonsäure, Naphthalin-1,5-disulfonsäure und Methansulfonsäure, Butansulfonsäure, Hexansulfonsäure sowie Imide, wie z.B. Phthalimid, Saccharin und Thiosaccharin.

Außerdem bevorzugt erfindungsgemäß zu verwendende Verbindungen sind auch Additionsprodukte aus Salzen von Metallen der I., II. und III. Hauptgruppe sowie des Zinns, sowie ferner Salze von Metallen der I., II., VII. und VIII. Nebengruppe des Periodensystems der Elemente und denjenigen 2-Aryl-substituierten 1,10-Phenanthrolinen der Formel (I), in denen $R^1$, $R^2$, $R^3$, $R^4$, $R^5$ und $R^6$ die Bedeutungen haben, die bereits im Zusammenhang mit der Beschreibung der erfindungsgemäß zu verwendenden Stoffe der Formel (I) vorzugsweise für diese Substituenten genannt wurden.

Hierbei sind Salze des Kupfers, Zinks, Mangans, Magnesiums, Calciums, Zinns, Eisens, Cobalts und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Im einzelnen seien außer den bei den Herstellungsbeispielen genannten Verbindungen die folgenden 2-Aryl-substituierten 1,10-Phenanthroline der allgemeinen Formel (I) genannt:

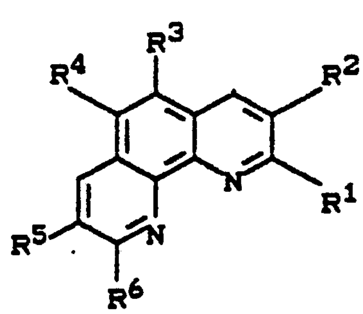

(I)

**Tabelle 1:**

| $R^1$ | $R^2$ | $R^3$ | $R^4$ | $R^5$ | $R^6$ |
|---|---|---|---|---|---|
| $CH_3$ | H | Cl | H | H | 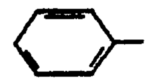 |
| $CH_3$ | H | H | $OCH_3$ | H | 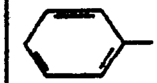 |
| $CH_3$ | H | $CH_3$ | H | H | 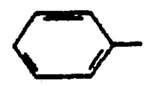 |
| $CH_3$ | H | $OCH_3$ | H | H | 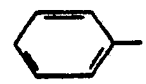 |
| $CH_3$ | H | Cl | H | $CH_3$ | 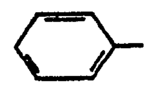 |
| $C_2H_5$ | $CH_3$ | Cl | Cl | H | 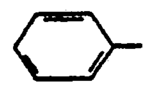 |
| $C_2H_5$ | H | $SCH_3$ | H | H | 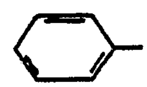 |
| 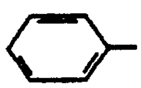 | H | $CH_3$ | $CH_3$ | H | 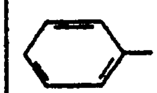 |
| 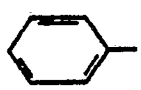 | $C_2H_5$ | Br | H | H | 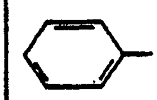 |
| H | H | H | $OCH_3$ | $CH_3$ | 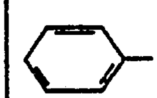 |
| 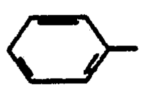 | H | H | H | H | 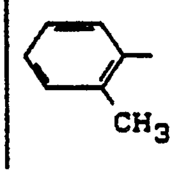 |

**Tabelle 1:** - Fortsetzung

| $R^1$ | $R^2$ | $R^3$ | $R^4$ | $R^5$ | $R^6$ |
|---|---|---|---|---|---|
| $CH_3$ | H | Br | H | H | 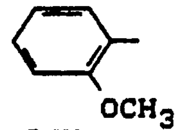 |
| H | $CH_3$ | Cl | Cl | H | 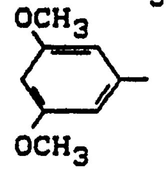 |
| $CH_3$ | H | H | $CH_3$ | H | 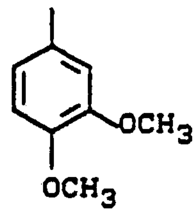 |
| $C_2H_5$ | $CH_3$ | H | H | $CH_3$ | 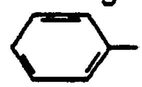 |
| 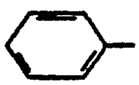 | H | $OCH_3$ | H | $C_2H_5$ | 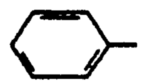 |
| 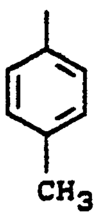 | H | $SCH_3$ | H | H | 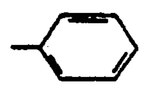 |
| 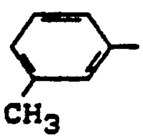 | $C_2H_5$ | Cl | H | H | 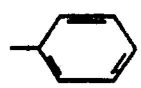 |
| 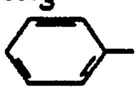 | H | H | $CH_3$ | H | 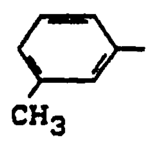 |
| $CH_3$ | H | H | $C_2H_5$ | H | 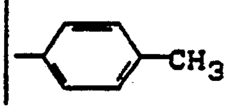 |

Tabelle 1: - Fortsetzung

| $R^1$ | $R^2$ | $R^3$ | $R^4$ | $R^5$ | $R^6$ |
|---|---|---|---|---|---|
| $CH_3$ | H | $SCH_3$ | H | H | 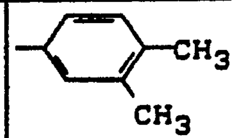 |
| H | $CH_3$ | H | Br | H | 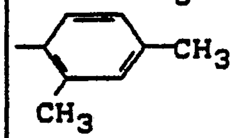 |
| $CH_3$ | H | Cl | H | H | 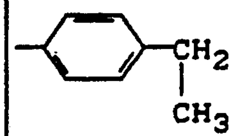 |
| $CH_3$ | H | H | $OC_2H_5$ | $C_2H_5$ | 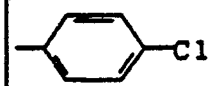 |
| $CH_3$ | H | H | $SC_2H_5$ | $CH_3$ | 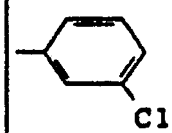 |
| H | $C_2H_5$ | $C_2H_5$ | H | H | 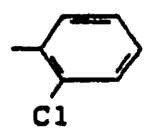 |
| $C_2H_5$ | $CH_3$ | H | H | H | 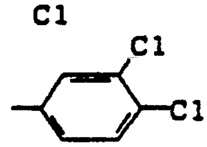 |
| 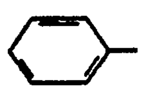 | H | $CH_3$ | $CH_3$ | H | 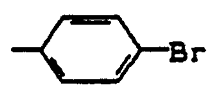 |
| $CH_3$ | H | Cl | H | H | 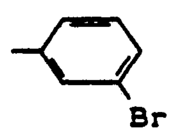 |

**Tabelle 1:** - Fortsetzung

| $R^1$ | $R^2$ | $R^3$ | $R^4$ | $R^5$ | $R^6$ |
|---|---|---|---|---|---|
| 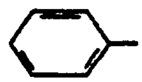 | H | Cl | H | H | 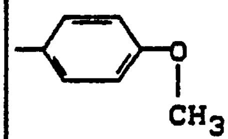 |
| $CH_3$ | H | H | Cl | H | 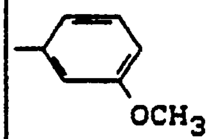 |
| $CH_3$ | H | H | H | H | 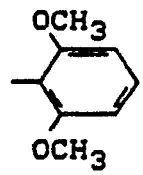 |
| $CH_3$ | H | H | $CH_3$ | H | 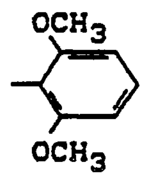 |
| H | $CH_3$ | Cl | H | H | 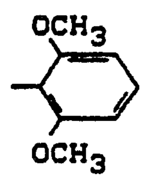 |

Weitere Beispiele für erfindungsgemäß zu verwendende 2-Aryl-substituierte 1,10-Phenanthroline der Formel (I) sind die Salze der in Tabelle (I) aufgeführten Verbindungen mit den obengenannten Säuren sowie die Metallsalz-Komplexe der in Tabelle (I) aufgeführten Verbindungen.

Hierbei sind Salze des Kupfers, Zinks, Mangans, Magnesiums, Calciums, Zinns, Eisens, Cobalts und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Ein weiterer Gegenstand der Anmeldung sind neue 2-Aryl-substituierte 1,10-Phenanthroline der Formel (Ia),

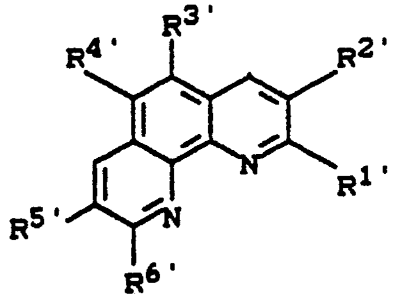

(Ia)

in welcher
$R^{1'}$ für Wasserstoff, Alkyl oder unsubstitiertes oder substituiertes Phenyl steht,
$R^{2'}$ and $R^{5'}$ gleich oder verschieden sind und für Wasserstoff oder Alkyl stehen,
$R^{3'}$ und $R^{4'}$ gleich oder verschieden sind und für Wasserstoff, Alkyl, Halogenalkyl, Halogen, Hydroxy, Alkoxy, Mercapto, Alkylthio, Alkylsulfinyl, Alkylsulfonyl, Nitro, unsubstituiertes oder substituiertes Aryl, Amino, Alkylamino, unsubstituiertes oder substituiertes Arylamino, Aralkylamino, Dialkylamino, Diaralkylamino oder für die Gruppierungen $-NH-CO-R^{7'}$, $-NH-CY'-X'-R^{8'}$, $-NH-SO_2-R^{9'}$, $-Y^{1'}-CO-R^{10'}$, $-Y^{2'}-CO-X^{1'}-R^{11'}$, $-Y^{3'}-CS-X^{2'}-R^{12'}$ oder $-CO-R^{13'}$ stehen,
worin
$R^{7'}$, $R^{8'}$, $R^{9'}$, $R^{10'}$, $R^{11'}$, $R^{12'}$ und $R^{13'}$ gleich oder verschieden sind und für Alkyl oder unsubstituiertes oder substituiertes Phenyl stehen,
$Y'$, $Y^{1'}$, $Y^{2'}$ und $Y^{3'}$ gleich oder verschieden sind und für Sauerstoff oder Schwefel stehen und
$X'$, $X^{1'}$ und $X^{2'}$ gleich oder verschieden sind und für Sauerstoff, Schwefel oder für die Gruppe $N-R^{14'}$ stehen, worin $R^{14'}$ für Wasserstoff oder Alkyl steht,
und
$R^{6'}$ für unsubstituiertes oder substituiertes Phenyl steht,
sowie deren Säureadditionssalze und Metallsalzkomplexe,
ausgenommen die Verbindungen 2,9-Bis-(4-methoxyphenyl)-1,10-phenanthrolin, 2,9-Diphenyl-1,10-phenanthrolin, 2,9-Bis-(p-hydroxyphenyl)-1,10-phenanthrolin, 2,9-Bis-[4-(2-propinyloxy)-phenyl]-1,10-phenanthrolin und 2-Phenyl-1,10-phenanthrolin,

Die noch nicht bekannten 2-Aryl-substituierten 1,10-Phenanthroline sind durch die Formel (Ia) allgemein definiert. Bevorzugt sind diejenigen Verbindungen der Formel (Ia), bei welchen
$R^{1'}$ für Wasserstoff, geradkettiges oder verzweigtes Alkyl mit 1 bis 4 Kohlenstoffatomen, unsubstituiertes oder einfach oder mehrfach, gleich oder verschieden durch Halogen, Alkyl, Alkoxy oder Halogenalkyl mit jeweils bis zu 4 Kohlenstoffatomen substituiertes Phenyl steht,
$R^{2'}$ und $R^{5'}$ gleich oder verschieden sind und für Wasserstoff oder geradkettiges oder verzweigtes Alkyl mit 1 bis 4 Kohlenstoffatomen stehen,
$R^{3'}$ und $R^{4'}$ gleich oder verschieden sind und für Wasserstoff, für geradkettiges oder verzweigtes Alkyl mit 1 bis 4 Kohlenstoffatomen, Halogenalkyl mit 1 oder 2 Kohlenstoffatomen und 1 bis 5 gleichen oder verschiedenen Halogenatomen, Halogen, Hydroxy, Alkoxy oder Alkylthio mit jeweils 1 bis 4 Kohlenstoffatomen, Mercapto, Alkylsulfinyl oder Alkylsulfonyl mit jeweils 1 bis 4 Kohlenstoffatomen, Nitro, unsubstituiertes oder einfach oder zweifach, gleich oder verschieden durch Halogen, Alkyl oder Halogenalkyl mit jeweils 1 bis 3 Kohlenstoffatomen und 1 bis 5 gleichen oder verschiedenen Halogenatomen substituiertes Phenyl, ferner für Amino, für jeweils geradkettiges oder verzweigtes Alkylamino oder Dialkylamino mit jeweils 1 bis 4 Kohlenstoffatomen je Alkylteil, unsubstituiertes oder einfach oder zweifach, gleich oder verschieden durch Halogen, Alkyl oder Halogenalkyl mit jeweils 1 bis 3 Kohlenstoffatomen und gegebenenfalls 1 bis 5 gleichen oder verschiedenen Halogenatomen substituiertes Phenylamino, für jeweils unsubstituiertes oder einfach bis mehrfach, gleich oder verschieden im Phenylteil durch Halogen substituiertes Phenylalkylamino oder Diphenylalkylamino mit jeweils 1 oder 2 Kohlenstoffatomen im Alkylteil oder für die Gruppierungen $-NH-CO-R^{7'}$, $-NH-CY'-X'-R^{8'}$, $-NH-SO_2-R^{9'}$, $Y^{1'}-CO-R^{10'}$, $-Y^{2'}-CO-X^{1'}-R^{11'}$, $-Y^{3'}-CS-X^{2'}-R^{12'}$ oder $-CO-R^{13'}$ stehen, worin
$R^{7'}$, $R^{8'}$, $R^{9'}$, $R^{10'}$, $R^{11'}$, $R^{12'}$ und $R^{13'}$ gleich oder verschieden sind und für geradkettiges oder verzweigtes Alkyl mit 1 bis 4 Kohlenstoffatomen oder für unsubstituiertes oder einfach bis dreifach, gleich oder verschieden durch Halogen, Nitro oder Alkyl mit 1 bis 4 Kohlenstoffatomen substituiertes Phenyl stehen,
$Y'$, $Y^{1'}$, $Y^{2'}$ und $Y^{3'}$ gleich oder verschieden sind und für Sauerstoff oder Schwefel stehen und
$X'$, $X^{1'}$ und $X^{2'}$ gleich oder verschieden sind und für Sauerstoff, Schwefel oder für die Gruppe $N-R^{14'}$

stehen, worin $R^{14'}$ für Wasserstoff oder geradkettiges oder verzweigtes Alkyl mit 1 bis 4 Kohlenstoffatomen steht, wobei, $R^3$ und $R^4$ insbesondere gleich oder verschieden sind und für Wasserstoff, jeweils geradkettiges oder verzweigtes Alkyl, Alkoxy oder Alkylthio mit 1 bis 4 Kohlenstoffatomen oder für Fluor, Chlor oder Brom stehen und
$R^6$ für unsubstituiertes oder einfach oder mehrfach, gleich oder verschieden durch Halogen, Alkyl, Alkoxy oder Halogenalkyl mit jeweils bis zu 4 Kohlenstoffatomen und gegebenenfalls 1 bis 5 gleichen oder verschiedenen Halogenatomen substituiertes Phenyl steht, ausgenommen die obengenannten Verbindungen.

Besonders bevorzugt sind diejenigen Verbindungen der Formel (I), in welchen
$R^1$ für Wasserstoff, Methyl, Ethyl, n- und i-Propyl, n-, i-, s- und t-Butyl, unsubstituiertes oder einfach bis dreifach, gleich oder verschieden durch Fluor, Chlor, Brom, Methyl, Ethyl, Methoxy, Ethoxy oder Trifluormethyl substituiertes Phenyl steht,
$R^2$ und $R^5$ gleich oder verschieden sind und für Wasserstoff, Methyl, Ethyl, n- oder i-Propyl, n-, i-, s-und t-Butyl stehen,
$R^3$ und $R^4$ gleich oder verschieden sind und für Wasserstoff, Methyl, Ethyl,n- und i-Propyl, Fluor, Chlor, Brom, Hydroxy, Methoxy, Ethoxy, n-Propoxy, i-Propoxy, Mercapto, Methylthio, Ethylthio, n-Butylsulfinyl, Methylsulfonyl, Nitro, unsubstituierten oder einfach oder zweifach, gleich oder verschieden durch Fluor, Chlor, Brom, Methyl, Ethyl, i-Propyl oder Trifluormethyl substituiertes Phenyl, ferner für Amino, Methylamino, Ethylamino, 2,6-Dichlorbenzylamino, Dimethylamino, Diethylamino, unsubstituiertes oder einfach oder zweifach, gleich oder verschieden durch Fluor, Chlor, Brom, Methyl, Ethyl, i-Propyl oder Trifluormethyl substituiertes Phenylamino oder für die Gruppierungen -NH-CO-$R^{7'}$, -NH-CY$'$-X$'$-$R^{8'}$, -NH-$SO_2R^{9'}$, -$Y^{1'}$-CO-$R^{10'}$, -$Y^{2'}$-CO-$X^{1'}$-$R^{11'}$, -$Y^{3'}$-CS-$X^{2'}$-$R^{12'}$ oder -CO-$R^{13'}$ stehen,
worin
$R^{7'}$, $R^{8'}$, $R^{9'}$, $R^{10'}$, $R^{11'}$, $R^{12'}$ und $R^{13'}$ gleich oder verschieden sind und für Methyl, Ethyl, n- und i-Propyl, n-, i-, s- und t-Butyl oder für gegebenenfalls einfach oder zweifach, gleich oder verschieden durch Fluor, Chlor, Brom, Nitro, Methyl, Ethyl substituiertes Phenyl stehen,
Y$'$, $Y^{1'}$, $Y^{2'}$ und $Y^{3'}$ gleich oder verschieden sind und für Sauerstoff oder Schwefel stehen und
X$'$, $X^{1'}$ und $X^{2'}$ gleich oder verschieden sind und für Sauerstoff, Schwefel oder für die Gruppe N-$R^{14'}$ stehen, worin
$R^{14'}$ für Wasserstoff oder Methyl steht, wobei
$R^3$ und $R^4$ insbesondere gleich oder verschieden sind und für Wasserstoff, Methyl, Ethyl, Methoxy, Ethoxy, Methylthio, Ethylthio, Fluor, Chlor oder Brom stehen und
$R^6$ für unsubstituierte oder einfach bis dreifach gleich oder verschieden durch Fluor, Chlor, Brom, Methyl, Ethyl, Methoxy, Ethoxy oder Trifluormethyl substituiertes Phenyl steht, ausgenommen die oben genannten Verbindungen.

Bevorzugte erfindungsgemäße Verbindungen sind auch Additionsprodukte aus Säuren und denjenigen 2-Aryl-substituierten 1,10-Phenanthrolinen der Formel (Ia) in denen $R^{1'}$, $R^{2'}$, $R^{3'}$, $R^{4'}$, $R^5$ und $R^6$ diejenigen Bedeutungen haben, die im Zusammenhang mit der Beschreibung der erfindungsgemäß zu verwendenden Stoffe bereits vorzugsweise für $R^1$, $R^2$, $R^3$, $R^4$, $R^5$ und $R^6$ genannt wurden, ausgenommen die oben angegebenen Verbindungen.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Malonsäure, Glutarsäure, Oxalsäure, Maleinsäure, Bernsteinsäure, Fumarsäure, Weinsäure, Zitronensäure, Salicylsäure, Sorbinsäure, und Milchsäure, Ölsäure, Palmitinsäure, Stearinsäure, unsubstituierte oder einfach oder mehrfach durch Nitro oder Halogen substituierte Benzoesäure, Gluconsäure, Ascorbinsäure, Äpfelsäure, Sulfamidsäure, Sulfonsäuren, wie z.B. Benzol-1,3-disulfonsäure, Benzolsulfonsäure, Perfluorbutansulfonsäure, p-Toluolsulfonsäure, 1,5- Naphthalindisulfonsäure und Methansulfonsäure, Butansulfonsäure, Hexansulfonsäure, sowie Imide wie z.B. Phthalimid, Saccharin und Thiosaccharin.

Außerdem bevorzugte erfindungsgemäße Verbindungen sind auch Additionsprodukte aus Salzen von Metallen der I., II. und III. Hauptgruppen sowie des Zinns, sowie ferner Salze von Metallen der I., II., VII. und VIII. Nebengruppe des Periodensystems der Elemente und denjenigen substituierten 1,10-Phenanthrolinen der Formel (Ia), in denen $R^{1'}$, $R^{2'}$, $R^{3'}$, $R^{4'}$, $R^5$ und $R^6$ die Bedeutungen haben, die bereits im Zusammenhang mit der Beschreibung der erfindungsgemäß zu verwendenden Stoffe der Formel (I) vorzugsweise für $R^1$, $R^2$, $R^3$, $R^4$, $R^5$ und $R^6$ genannt wurden, ausgenommen die oben genannten Verbindungen.

Hierbei sind Salze des Kupfers, Zinks, Mangans, Magnesiums, Calciums, Zinns, Eisens, Cobalts und

des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Man erhält die neuen 2-Aryl-substituierten 1,10-Phenanthroline der Formel (Ia),

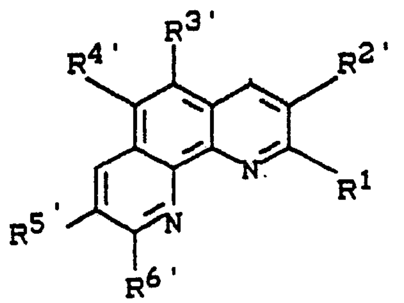

(Ia)

in welcher
$R^{1'}$ für Wasserstoff, Alkyl oder unsubstituiertes oder substituiertes Phenyl steht,
$R^{2'}$ und $R^{5'}$ gleich oder verschieden sind und für Wasserstoff oder Alkyl stehen,
$R^{3'}$ und $R^{4'}$ gleich oder verschieden sind und für Wasserstoff, Alkyl, Halogenalkyl, Halogen, Hydroxy, Alkoxy, Mercapto, Alkylthio, Alkylsulfinyl, Alkylsulfonyl, Nitro, unsubstituiertes oder substituiertes Aryl, Amino, Alkylamino, unsubstituiertes oder substituiertes Arylamino, Aralkylamino, Dialkylamino, Diaralkylamino oder für die Gruppierungen $-NH-CO-R^{7'}$, $-NH-CY'-X'-R^{8'}$, $-NH-SO_2-R^{9'}$, $-Y^{1'}-CO-R^{10'}$, $-Y^{2'}-CO-X^{1'}-R^{11'}$, $-Y^{3'}-CS-X^{2'}-R^{12'}$ oder $-CO-R^{13'}$ stehen,
worin
$R^{7'}$, $R^{8'}$, $R^{9'}$, $R^{10'}$, $R^{11'}$, $R^{12'}$ und $R^{13'}$ gleich oder verschieden sind und für Alkyl oder unsubstituiertes oder substituiertes Phenyl stehen,
$Y'$, $Y^{1'}$, $Y^{2'}$ und $Y^{3'}$ gleich oder verschieden sind und für Sauerstoff oder Schwefel stehen und
$X'$, $X^{1'}$ und $X^{2'}$ gleich oder verschieden sind und für Sauerstoff, Schwefel oder für die Gruppe $N-R^{14'}$ stehen, worin $R^{14'}$ für Wasserstoff oder Alkyl steht,
und
$R^{6'}$ für unsubstituiertes oder substituiertes Phenyl steht, sowie deren Säureadditionssalze und Metallsalzkomplexe,
ausgenommen die Verbindungen 2,9-Bis-(4-methoxyphenyl)-1,10-phenanthrolin, 2,9-Diphenyl-1,10-phenanthrolin, 2,9-Bis-(p-hydroxyphenyl)-1,10-phenanthrolin, 2,9-Bis-[4-(2-propinyloxy)-phenyl]-1,10-phenanthrolin und 2-Phenyl-1,10-phenanthrolin, wenn man
A) 8-Aminochinoline der Formel (II)

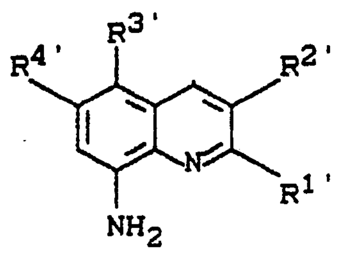

(II)

in welcher
$R^{1'}$, $R^{2'}$, $R^{3'}$ und $R^{4'}$ die oben angegebene Bedeutung haben,
mit α,ß-ungesättigten Aldehyden der Formel (III),

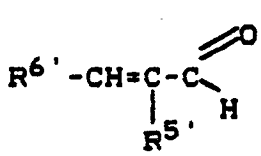

(III)

in welcher ,
$R^5$ und $R^6$ die oben angegebene Bedeutung haben,
gegebenenfalls in Gegenwart von Verdünnungsmitteln sowie gegebenenfalls in Gegenwart einer starken Säure oder gegebenenfalls in Gegenwart eines Oxidationsmittels oder unter Wasserabscheidung gegebenenfalls in Gegenwart eines Katalysators cyclisiert und die so erhaltenen Produkte gegebenenfalls in Säureadditions-Salze und Metallsalz-Komplexe überführt,
oder wenn man
B) substituierte 1,10-Phenanthrolin-Derivate der Formel (Ib),

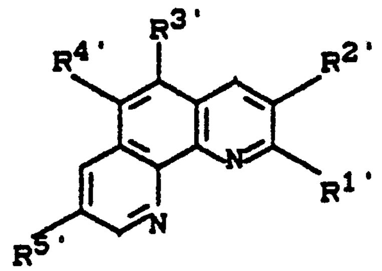

(Ib)

in welcher
$R^1$, $R^2$, $R^3$, $R^4$ und $R^5$ die oben angegebene Bedeutung haben,
in allgemein üblicher Art und Weise
α) mit Lithiumverbindungen der Formel (IV)
$R^6$ - Li (IV)
in welcher
$R^6$ die oben angegebene Bedeutung hat,
oder
ß) mit Magnesium-organischen Verbindungen der Formel (IVa)
$R^6$ - Mg - X (IVa)
in welcher
$R^6$ die oben angegebene Bedeutung hat und
X für Chlor, Brom oder Iod, vorzugsweise für Brom, steht,
gegebenenfalls in Gegenwart von Verdünnungsmitteln und gegebenenfalls in Gegenwart eines Inertgases umsetzt, anschließend hydrolysiert, danach mit einem Oxidationsmittel dehydriert und die so erhaltenen Produkte gegebenenfalls in deren Säureadditions-Salze oder Metallsalz-Komplexe überführt.

Die bekannten Verbindungen der Formel (I) lassen sich analog zu den oben genannten Verfahren zur Herstellung der neuen Verbindungen der Formel (Ia) herstellen.

Verwendet man als Ausgangsstoffe beispielsweise 8-Amino-chinaldin und Zimtaldehyd und Aluminiumchlorid als Lewis-Säure, so läßt sich der Reaktionsablauf des Herstellungsverfahrens (A) durch das folgende Formelschema darstellen:

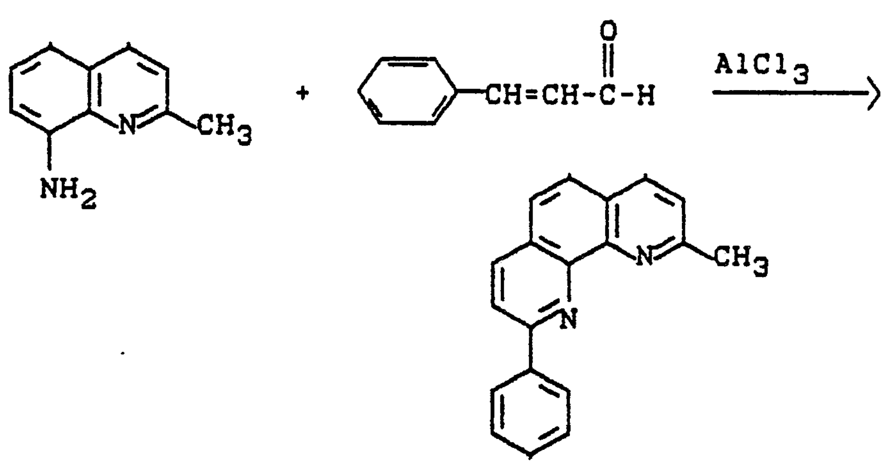

Verwendet man als Ausgangsstoffe beispielsweise 1,10-Phenanthrolin, Phenyllithium und Braunstein, so läßt sich der Reaktionsablauf des Herstellungsverfahrens (B/α) durch das folgende Formelschema darstellen:

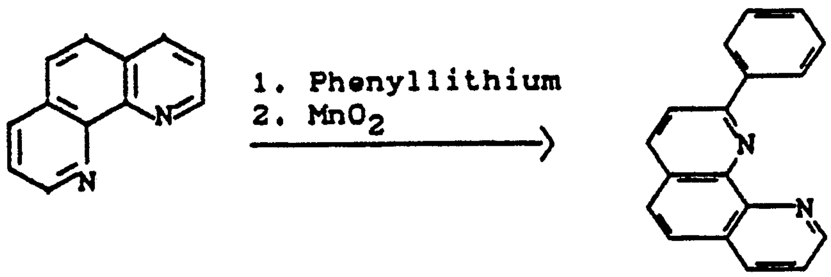


Verwendet man als Ausgangsstoffe beispielsweise 1,10-Phenanthrolin, Phenylmagnesiumbromid und Braunstein, so läßt sich der Reaktionsablauf des Herstellungsverfahrens (B/ß) durch das folgende Formelschema darstellen:

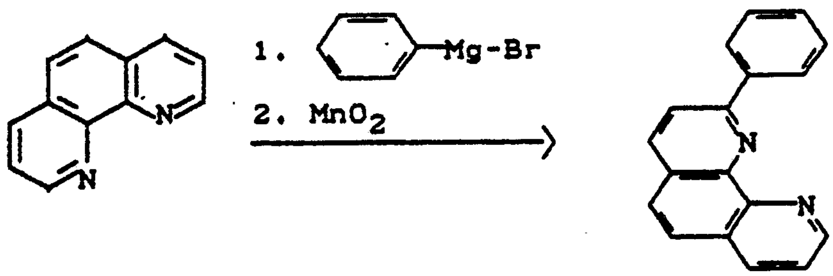


Die zur Durchführung der Herstellungsverfahren (A) als Ausgangsstoffe benötigten 8-Aminochinoline sind durch die Formel (II) allgemein definiert. In dieser Formel (II) stehen $R^1$, $R^2$, $R^3$ und $R^4$ vorzugsweise bzw. insbesondere für diejenigen Substituenten, die oben bei der Beschreibung der neuen 1,10-Phenanthroline der Formel (Ia) als bevorzugt bzw. besonders bevorzugt für diese Reste genannt wurden.

Die 8-Aminochinoline der Formel (II) sind bekannt und/oder können nach bekannten Verfahren in einfacher, analoger Weise hergestellt werden (vgl. z.B. Org. Reactions 7, 59 ff., (1953); Kirk-Othmer, 2. Aufl., 15, 869), indem man beispielsweise die bekannten 2-Nitroaniline der Formel (V),

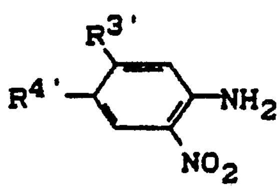
(V)

in welcher,
$R^3$ und $R^4$ die oben angegebene Bedeutung haben,
mit den entsprechenden Aldehyden gegebenenfalls in Gegenwart eines Verdünnungsmittels und gegebenenfalls in Gegenwart eines Katalysators sowie gegebenenfalls in Gegenwart eines Oxidationsmittels, bei Temperaturen zwischen 20 °C und 180 °C zunächst zu den 8-Nitro-chinolinen der Formel (IIa),

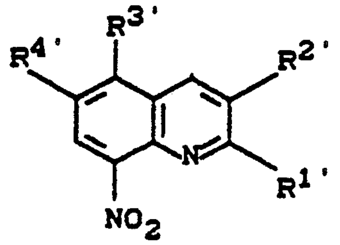

(IIa)

in welcher

$R^{1'}$, $R^{2'}$, $R^{3'}$ und $R^{4'}$ die oben angegebene Bedeutung haben,

cyclisiert und dann die Nitrogruppe gegebenenfalls in Gegenwart eines Verdünnungsmittels in üblicher Art und Weise und in Gegenwart eines Katalysators, wie bei spielsweise Raney-Nickel oder Zinn-II-Salzen, bei Temperaturen zwischen 20 °C und 150 °C und gegebenenfalls unter Druck zwischen 2 und 100 bar reduziert.

Die weiterhin zur Durchführung des Herstellungsverfahrens (A) als Ausgangsstoffe benötigten Aldehyde sind durch die Formel (III) allgemein definiert. In der Formel (III) haben die Reste $R^5$ und $R^6$ vorzugsweise bzw. insbesondere diejenige Bedeutung, die oben im Zusammenhang mit der Beschreibung der erfindungsgemäßen Stoffe der Formel (Ia) als bevorzugt bzw. besonders bevorzugt angegeben ist.

Die Verbindungen der Formel (III) sind allgemein bekannte Verbindungen der organischen Chemie.

Die zur Durchführung des Herstellungsverfahrens (B) als Ausgangsstoffe benötigten 2-Aryl-substituierten 1,10-Phenanthrolin-Derivate sind durch die Formel (Ib) allgemein definiert. In dieser Formel (Ib) stehen $R^1$, $R^2$, $R^3$, $R^4$ und $R^5$ vorzugsweise bzw. insbesondere für diejenigen Substituenten, die schon bei der Beschreibung der neuen 2-Aryl-substituierten 1,10-Phenanthroline der Formel (Ia) als bevorzugt bzw. besonders bevorzugt für diese Reste genannt wurden. Die substituierten 1,10-Phenanthrolin-Derivate der Formel (Ib) sind teilweise bekannt (vgl. DE-OS 37 09 263) und/oder können nach dem Herstellungsverfahren (A) erhalten werden.

Die weiterhin zur Durchführung des Herstellungsverfahrens (B) benötigten Lithium- und Magnesium-organischen Verbindungen sind durch die Formeln (IV) und (IVa) allgemein definiert. In diesen Formeln (IV) und (IVa) steht $R^6$ vorzugsweise bzw. insbesondere für diejenige Bedeutung, die oben bei der Beschreibung der neuen 2-Aryl-substituierten 1,10-Phenanthroline der Formel (Ia) als bevorzugt bzw. besonders bevorzugt für $R^6$ genannt wurde.

Die Lithium- und Magnesium-organischen Verbindungen der Formeln (IV) und (IVa) sind allgemein bekannte Verbindungen der organischen Chemie.

Als Verdünnungsmittel zur Durchführung des Herstellungsverfahrens (A) kommen Mischungen von starken anorganischen oder organischen Säuren mit Wasser oder inerten organischen Lösungsmitteln infrage.

Säuren, welche zur Durchführung des Herstellungsverfahrens (A) verwendet werden können sind beispielswiese Schwefelsäure, Salzsäure und p-Toluolsulfonsäure und Lewis-Säuren, wie beispielsweise Aluminiumchlorid und Eisen-(III)-chlorid, insbesondere Aluminiumchlorid.

Zur Durchführung des Herstellungsverfahrens (A) können praktisch alle inerten organischen Lösungsmittel verwendet werden, insbesondere aliphatische und aromatische, gegebenenfalls halogenierte Kohlenwasserstoffe, wie beispielsweise, Benzin, Benzol, Toluol, Xylol, Chlorbenzol, Dichlorbenzol, Petrolether, Pentan, Hexan, Heptan, Ligroin, Cyclohexan, Dichlormethan oder Dichlorethan.

Die Reaktionstemperaturen können bei der Durchführung des Herstellungsverfahrens (A) in einem größeren Bereich variiert werden. Im allgemeinen arbeitet man bei Temperaturen zwischen 20 °C und 180 °C, vorzugsweise bei Temperaturen zwischen 40 °C und 130 °C.

Als Oxidationsmittel zur Durchführung des Herstellungsverfahrens (A) kommen die für derartige Reaktionen üblichen Oxidationsmittel infrage; vorzugsweise verwendet man Arsensäure oder Nitrogruppen enthaltende aromatische Säuren oder deren Alkalimetall-Salze, wie beispielsweise 3-Nitrobenzolsulfonsäure.

Zur Durchführung des Herstellungsverfahrens (A) setzt man pro Mol 8-Aminochinolin der Formel (II) 1,0 bis 2,0 Mol, vorzugsweise 1,0 bis 1,5 Mol an Aldehyden der Formel (III) und gegebenenfalls 0,5 bis 1,0 Mol, vorzugsweise 1,0 Mol, Oxidationsmittel oder Lewis-Säuren ein. Die Reaktionsführung, Aufarbeitung und Isolierung der Reaktionsprodukte der Formel (I) erfolgt in allgemein üblicher Art und Weise.

Als Verdünnungsmittel zur Durchführung der Herstellungsverfahren (B/α) und (B/ß) kommen praktisch alle inerten organischen Lösungsmittel infrage. Hierzu gehören vorzugsweise aromatische Kohlenwasserstoffe wie Pentan, Hexan, Heptan, Cyclohexan, Petrolether, Benzin, Ligroin, Benzol, Toluol und Xylol und

Ether, wie Diethylether, Dioxan, Tetrahydrofuran oder Ethylenglykoldimethyl- oder -diethylether.

Die Reaktionstemperaturen können bei der Durchführung der Herstellungsverfahren (B/α) und (B/ß) in einem größeren Bereich variiert werden. Im allgemeinen arbeitet man bei Temperaturen zwischen -40 °C und +40 °C, vorzugsweise bei Temperaturen zwischen 0 °C und 40 °C.

Als Inertgase kommen für die Durchführung der Herstellungsverfahren (B/α) und (B/ß) Stickstoff sowie praktisch alle Edelgase, insbesondere Argon infrage.

Zur Durchführung des Herstellungsverfahrens (B/α) setzt man pro Mol 1,10-Phenanthrolin der Formel (Ib) im allgemeinen 1,0 bis 6,0 Mol vorzugsweise 2,0 bis 4,0 Mol Lithiumverbindung der Formel (IV) und 20,0 bis 100 Mol, vorzugsweise 20,0 bis 50,0 Mol an Oxidationsmittel ein.

Die Reaktionen werden im allgemeinen in einem geeigneten Verdünnungsmittel durchgeführt und das Reaktionsgemisch wird mehrere Stunden bei der jeweils erforderlichen Temperatur gerührt. Anschließend wird hydrolysiert und mit einem geeigneten Oxidationsmittel, vorzugsweise Braunstein, dehydriert. Die Aufarbeitung erfolgt jeweils nach üblichen Methoden.

Zur Durchführung des Herstellungsverfahrens (B/ß) setzt man pro Mol 1,10-Phenanthrolin der Formel (Ib) im allgemeinen 1,0 bis 10,0 Mol, vorzugsweise 2,0 bis 5,0 Mol an Magnesium-organischen Verbindungen der Formel (IVa) und 20,0 bis 100 Mol, vorzugsweise 20,0 bis 50,0 Mol an Oxidationsmittel ein.

Die Reaktionen werden im allgemeinen in einem geeigneten Verdünnungsmittel durchgeführt und das Reaktionsgemisch wird mehrere Stunden bei der jeweils erforderlichen temperatur gerührt. Anschließend wird hydrolysiert und mit einem geeigneten Oxdidationsmittel, vorzugsweise Braunstein, dehydriert. Die Aufarbeitung erfolgt jeweils nach üblichen Methoden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen in Frage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol, und Hinzufügen zu Verbindungen der allgemeinen Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke biologische Wirkung auf und können zur Bekämpfung von unerwünschten Schädlingen praktisch eingesetzt werden. Die Wirkstoffe sind z. B. für den Gebrauch als Pflanzenschutzmittel einsetzbar, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);

Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg protektiv zur Bekämpfung von Botrytis-Arten bei Bohnen, Leptosphaeria-Arten an Weizen und Pyrenophora-Arten an Gerste eingesetzt werden.

Darüber hinaus zeigen die erfindungsgemäßen Wirkstoffe auch eine gute Wirkung gegen Cochliobolus sativus, Pyricularia oryzae, Pellicularia sasakii sowie eine gute und breite Wirkung im Agarplattentest.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwas serstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung und die Verwendung der erfindungsgemäßen Wirkstoffe geht aus den folgenden Beispielen hervor.

Herstellungsbeispiele

Beispiel 1

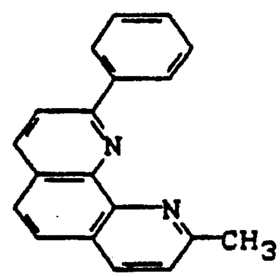

15.8 g (0,1 Mol) 8-Aminochinaldin und 13,4 g (0,1 Mol) Aluminiumchlorid werden in 150 ml trockenem Methylenchlorid 30 Minuten unter Rückfluß zum Sieden erhitzt. Danach werden 13,2 g (0,1 Mol) Zimtaldehyd in 15 ml Methylenchlorid innerhalb von 1 Stunde zugetropft und weitere 8 Stunden unter Rückfluß erhitzt. Nach dem Abkühlen auf Raumtemperatur wird unter vermindertem Druck eingeengt, der Rückstand mit Wasser aufgenommen und mit Methylenchlorid ausgeschüttelt. Die organische Phase wird mit Natriumsulfat getrocknet und über 100 g Aluminiumoxid filtriert. Das Filtrat wird unter vermindertem Druck eingeengt, der Rückstand mit Ether verrührt, abgesaugt, erneut mit Ether gewaschen und getrocknet. Der Feststoff wird mit 50 ml Toluol erhitzt, filtriert, das Filtrat unter vermindertem Druck eingeengt und der Rückstand aus 50 ml Ligroin umkristallisiert.

Man erhält 2,3 g (25 % der Theorie) 2-Methyl-9-phenyl-1,10-phenanthrolin vom Schmelzpunkt 145-9° C.

Beispiel 2

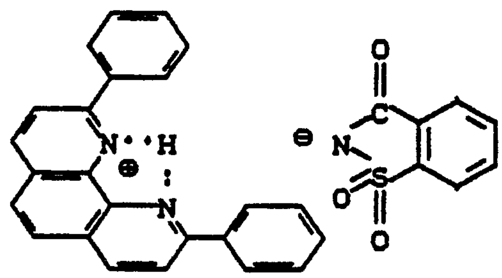

2,5 g (0,0075 Mol) 2,9-Diphenyl-1,10-phenanthrolin und 1,4 g (0,0075 Mol) Saccharin werden in je 50 ml wasserfreiem Ethanol gelöst, die Lösungen vereinigt und 5 Minuten unter Rückfluß zum Sieden erhitzt. Nach dem Einengen unter vermindertem Druck fällt ein viskoses Öl an.
Man erhält 3,0 g (77 % der Theorie) des Saccharin-Salzes des 2,9-Diphenyl-1,10-phenanthrolins.
($^1$H-NMR $\delta$ = 7,8 (s, 2H); 8,4-8,5 (m, 4H)*

(*) Die $^1$H-NMR-Spektren wurden in Deuterochloroform ($CDCl_3$) mit Tetramethylsilan (TMS) als innerem Standard aufgenommen. Angegeben ist die chemische Verschiebung als $\delta$-Wert in ppm.)

In analoger Weise zu den in den Beispielen 1 und 2 beschriebenen Methoden und unter Berücksichtigung der Angaben in den Beschreibungen zu den erfindungsgemäßen Verfahren werden die nachfolgend aufgeführten Endprodukte der Formel (I),

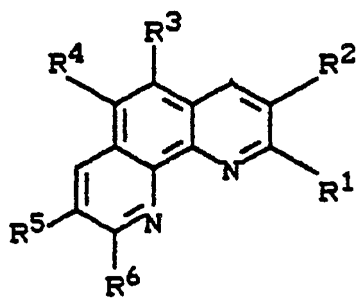

(I)

erhalten:

| Bsp. Nr. | $R^1$ | $R^2$ | $R^3$ | $R^4$ | $R^5$ | $R^6$ | Metallkomplex, Säureadditions-Salz | Schmelzpunkt° C |
|---|---|---|---|---|---|---|---|---|
| 3 | 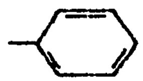 | H | Cl | H | H | $CH_3$ | $ZnCl_2$ | 285-9 |
| 4 | $CH_3$ | H | H | Cl | H | 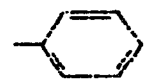 | | 192-6 |
| 5 | 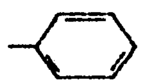 | H | H | H | H | 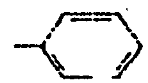 | | 185-6 |
| 6 | 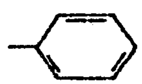 | H | H | H | H | 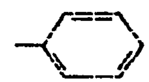 | $CuCl_2$ x1/2 $C_2H_5OH$ | 240 |
| 7 | 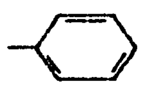 | H | H | H | H | H | | $\delta$ = 9,3 (dd,1H)* |
| 8 | $CH_3$ | H | H | H | $C_2H_5$ | 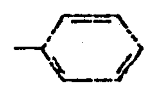 | | 176 |

| Bsp. Nr. | $R^1$ | $R^2$ | $R^3$ | $R^4$ | $R^5$ | $R^6$ | Metallkomplex, Säureadditions-Salz | Schmelzpunkt° C |
|---|---|---|---|---|---|---|---|---|
| 9 | $CH_3$ | H | H | H | $C_2H_5$ | 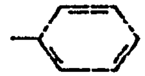 | $CuCl_2$ | 213 (Zers.) |
| 10 | H | H | H | H | H | 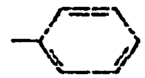 | $CuCl_2$ x1/2 $H_2O$ | 239 |
| 11 | $CH_3$ | H | H | H | $C_2H_5$ | 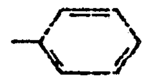 | $ZnCl_2$ | > 300 |
| 12 | $CH_3$ | H | H | H | $C_2H_5$ | 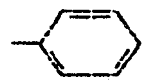 | Apfelsäure | S = 4,3 (m,1H)** |
| 13 | 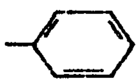 | H | H | H | H | H | 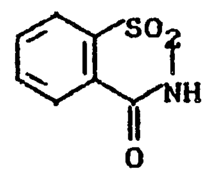 | 214 |

*) Die $^1$H-NMR-Spektren wurden in Deuterochloroform ($CDCl_3$) und (DMSO)** mit Tetramethylsilan (TMS) als innerem Standard aufgenommen. Angegehen ist die chemische Verschiebung als δ-Wert in ppm.

Anwendungsbeispiele:

In den folgenden Anwendungsbeispielen werden die nachstehend aufgeführten Verbindungen als Vergleichssubstanzen eingesetzt:

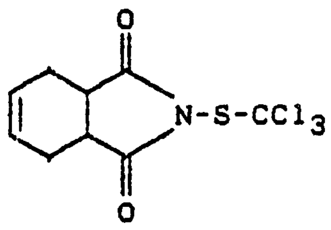

(A)

cis-N-((Trichlormethyl)thio)-4-cyclohexen-1,2-dicarboimid
(bekannt aus Science, (Washington) 115, 84 (1952); US-PS 2 553 770).

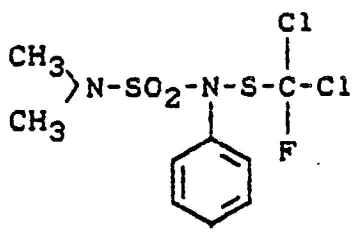

(B)

N,N-Dimethyl-N′-phenyl-N′-(fluordichlormethylthio)sulfamid
(bekannt aus DAS 1 193 498).

Beispiel A

Botrytis-Test (Bohnen)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

In diesem Test zeigt z.B. die erfindungsgemäße Verbindung (1) eine bessere Wirksamkeit als die Vergleichssubstanz (B).

**Tabelle A**

Botrytis-Test (Buschbohnen)/protektiv

| Wirkstoff | Wirkungsgrad in % bei einer Wirkstoffkonzentration von 50 ppm |
|---|---|
| 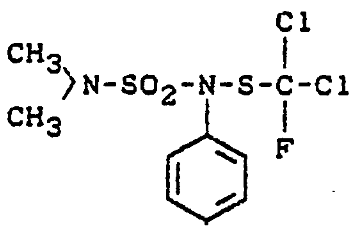 (bekannt) (B) | 54 |
| 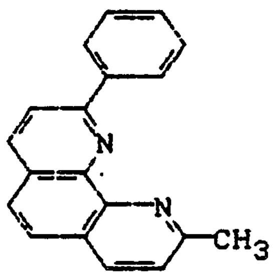 (1) | 96 |

Beispiel B

Leptosphaeria nodorum-Test (Weizen)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

In diesem Test zeigt z.B. die erfindungsgemäße Verbindung (1) eine bessere Wirksamkeit als die Vergleichssubstanz (A).

## Tabelle B

Leptosphaeria nodorum-Test (Weizen) / protektiv

| Wirkstoff | Wirkstoff-konzentration in der Spritzbrühe in Gew.-% | Wirkungsgrad in % der unbehandelten Kontrolle |
|---|---|---|
| 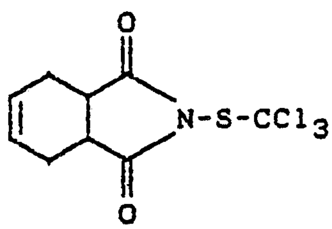 (bekannt) (A) | 0,025 | 27 |
| 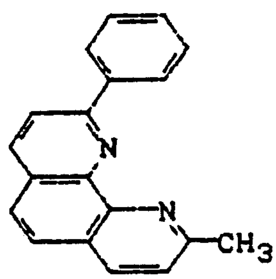 (1) | 0,025 | 100 |

Beispiel C

Pyrenophora teres-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

In diesem Test zeigt z.B. die erfindungsgemäße Verbindung (1) eine bessere Wirksamkeit als die Vergleichssubstanz (A).

## Tabelle C

Pyrenophora teres-Test (Gerste) / protektiv

| Wirkstoff | Wirkstoff-konzentration in der Spritzbrühe in Gew.-% | Wirkungsgrad in % der unbehandelten Kontrolle |
|---|---|---|
| 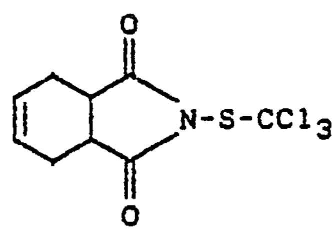 (bekannt) (A) | 0,025 | 38 |
| 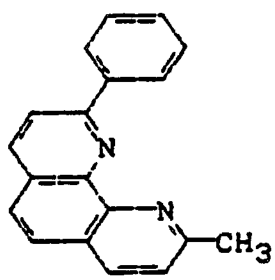 (1) | 0,025 | 100 |

**Ansprüche**

1. Verwendung von 2-Aryl-substituierten 1,10-Phenanthrolinen der allgemeinen Formel (I),

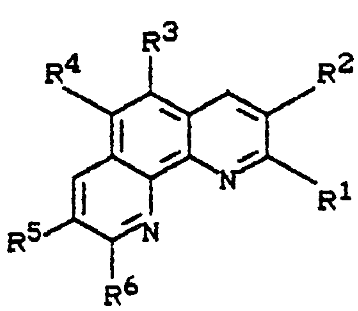

(I)

in welcher
$R^1$ für Wasserstoff, Alkyl oder unsubstituiertes oder substituiertes Phenyl steht,
$R^2$ und $R^5$ gleich oder verschieden sind und für Wasserstoff oder Alkyl stehen,
$R^3$ und $R^4$ gleich oder verschieden sind und für Wasserstoff, Alkyl, Halogenalkyl, Halogen, Hydroxy, Alkoxy, Mercapto, Alkylthio, Alkylsulfinyl, Alkylsulfonyl, Nitro, unsubstituiertes oder substituiertes Aryl, Amino, Alkylamino, unsubstituiertes oder substituiertes Arylamino, Aralkylamino, Dialkylamino, Diaralkylamino oder

für die Gruppierungen $-NH-CO-R^7$, $-NH-CY-X-R^8$, $-NH-SO_2-R^9$, $-Y^1-CO-R^{10}$, $-Y^2-CO-X^1-R^{11}$, $-Y^3-CS-X^2-R^{12}$ oder $-CO-R^{13}$ stehen,
worin
$R^7$, $R^8$, $R^9$, $R^{10}$, $R^{11}$, $R^{12}$ und $R^{13}$ gleich oder verschieden sind und für Alkyl oder unsubstituiertes oder substituiertes Phenyl stehen,
Y, $Y^1$, $Y^2$ und $Y^3$ gleich oder verschieden sind und für Sauerstoff oder Schwefel stehen und
X, $X^1$ und $X^2$ gleich oder verschieden sind und für Sauerstoff, Schwefel oder für die Gruppe $N-R^{14}$ stehen, worin $R^{14}$ für Wasserstoff oder Alkyl steht,
und
$R^6$ für unsubstituiertes oder substituiertes Phenyl steht,
sowie deren Säureadditions-Salze und Metallsalz-Komplexe zur Bekämpfung von Schädlingen.

2. 2-Aryl-substituierte 1,10-Phenanthroline der Formel (Ia),

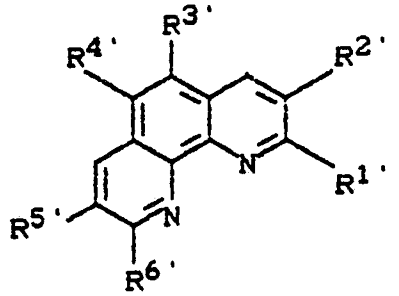

(Ia)

in welcher
$R^1$, für Wasserstoff, Alkyl oder unsubstitiertes oder substituiertes Phenyl steht,
$R^2$, und $R^5$ gleich oder verschieden sind und für Wasserstoff oder Alkyl stehen,
$R^3$ und $R^4$ gleich oder verschieden sind und für Wasserstoff, alkyl, Halogenalkyl, Halogen, Hydroxy, Alkoxy, Mercapto, Alkylthio, Alkylsulfinyl, Alkylsulfonyl, Nitro, unsubstituiertes oder substituiertes Aryl, Amino, Alkylamino, unsubstituiertes oder substituiertes Arylamino, Aralkylamino, Dialkylamino, Diaralkylamino ,oder für die Gruppierungen $-NH-CO-R^7$, $-NH-CY-X-R^8$, $-NH-SO_2-R^9$, $-Y^1-CO-R^{10}$, $-Y^2-CO-X^1-R^{11}$, $-Y^3-CS-X^2-R^{12}$ oder $-CO-R^{13}$ stehen,
worin
$R^7$, $R^8$, $R^9$, $R^{10}$, $R^{11}$, $R^{12}$ und $R^{13}$ gleich oder verschieden sind und für Alkyl oder unsubstituiertes oder substituiertes Phenyl stehen,
Y, $Y^1$, $Y^2$ und $Y^3$ gleich oder verschieden sind und für Sauerstoff oder Schwefel stehen und
X, $X^1$ und $X^2$ gleich oder verschieden sind und für Sauerstoff, Schwefel oder für die Gruppe $N-R^{14}$ stehen, worin $R^{14}$ für Wasserstoff oder Alkyl steht,
und
$R^6$ für unsubstituiertes oder substituiertes Phenyl steht,
sowie deren Säureadditionssalze und Metallsalzkomplexe,
ausgenommen die Verbindungen 2,9-Bis-(4-methoxyphenyl)-1,10-phenanthrolin, 2,9-Diphenyl-1,10-phenanthrolin, 2,9-Bis-(p-hydroxyphenyl)-1,10-phenanthrolin, 2,9-Bis-[4-(2-propinyloxy)-phenyl]-1,10-phenanthrolin und 2-Phenyl-1,10-phenanthrolin.

3. 2-Aryl-substituierte 1,10-Phenanthroline gemäß Anspruch 2, wobei in der Formel (Ia)
$R^1$ für Wasserstoff, geradkettiges oder verzweigtes Alkyl mit 1 bis 4 Kohlenstoffatomen, unsubstituiertes oder einfach oder mehrfach, gleich oder verschieden durch Halogen, Alkyl, Alkoxy oder Halogenalkyl mit jeweils bis zu 4 Kohlenstoffatomen substituiertes Phenyl steht,
$R^2$ und $R^5$ gleich oder verschieden sind und für Wasserstoff oder geradkettiges oder verzweigtes Alkyl mit 1 bis 4 Kohlenstoffatomen stehen,
$R^3$ und $R^4$ gleich oder verschieden sind und für Wasserstoff, für geradkettiges oder verzweigtes Alkyl mit 1 bis 4 Kohlenstoffatomen, Halogenalkyl mit 1 oder 2 Kohlenstoffatomen und 1 bis 5 gleichen oder verschiedenen Halogenatomen, Halogen, Hydroxy, Alkoxy oder Alkylthio mit jeweils 1 bis 4 Kohlenstoffatomen, Mercapto, Alkylsulfinyl oder Alkylsulfonyl mit jeweils 1 bis 4 Kohlenstoffatomen, Nitro, unsubstituiertes oder einfach oder zweifach, gleich oder verschieden durch Halogen, Alkyl oder Halogenalkyl mit jeweils 1 bis 3 Kohlenstoffatomen und 1 bis 5 gleichen oder verschiedenen Halogenatomen substituiertes Phenyl, ferner für Amino, für jeweils geradkettiges oder verzweigtes Alkylamino oder Dialkylamino mit jeweils 1 bis

4 Kohlenstoffatomen je Alkylteil, unsubstituiertes oder einfach oder zweifach, gleich oder verschieden durch Halogen, Alkyl oder Halogenalkyl mit jeweils 1 bis 3 Kohlenstoffatomen und unsubstituiertes oder 1 bis 5 gleichen oder verschiedenen Halogenatomen substituiertes Phenylamino, für jeweils gegebenenfalls einfach bis mehrfach, gleich oder verschieden im Phenylteil durch Halogen substituiertes Phenylalkylamino oder Diphenylalkylamino, mit jeweils 1 oder 2 Kohlenstoffatomen im Alkylteil oder für die Gruppierungen -NH-CO-$R^7$, -NH-CY'-X'-$R^8$, -NH-$SO_2$-$R^9$, -$Y^1$-CO-$R^{10}$, -$Y^2$-CO-$X^1$-$R^{11}$, -$Y^3$-CS-$X^2$-$R^{12}$ oder -CO-$R^{13}$ stehen,
worin
$R^7$, $R^8$, $R^9$, $R^{10}$, $R^{11}$, $R^{12}$ und $R^{13}$ gleich oder verschieden sind und für geradkettiges oder verzweigtes Alkyl mit 1 bis 4 Kohlenstoffatomen oder für unsubstituiertes oder oder einfach bis dreifach, gleich oder verschieden durch Halogen, Nitro oder Alkyl mit 1 bis 4 Kohlenstoffatomen substituiertes Phenyl stehen,
Y, $Y^1$, $Y^2$ und $Y^3$ gleich oder verschieden sind und für Sauerstoff oder Schwefel stehen und
X', $X^1$ und $X^2$ gleich oder verschieden sind und für Sauerstoff, Schwefel oder für die Gruppe N-$R^{14}$ stehen, worin $R^{14}$ für Wasserstoff oder geradkettiges oder verzweigtes Alkyl mit 1 bis 4 Kohlenstoffatomen steht, und
$R^6$ für unsubstituiertes oder einfach oder mehrfach, gleich oder verschieden durch Halogen, Alkyl, Alkoxy oder Halogenalkyl mit jeweils bis zu 4 Kohlenstoffatomen und gegebenenfalls 1 bis 5 gleichen oder verschiedenen Halogenatomen substituiertes Phenyl steht.

4. 2-Aryl-substituierte 1,10-Phenanthroline gemäß Anspruch 2, wobei in der Formel (Ia)
$R^1$ für Wasserstoff, Methyl, Ethyl, n- und i-Propyl, n-, i-, s- und t-Butyl, unsubstituiertes oder einfach bis dreifach, gleich oder verschieden durch Fluor, Chlor, Brom, Methyl, Ethyl, Methoxy, Ethoxy oder Trifluormethyl substituiertes Phenyl steht,
$R^2$ und $R^5$ gleich oder verschieden sind und für Wasserstoff, Methyl, Ethyl, n- oder i-Propyl, n-, i-, s- und t-Butyl stehen,
$R^3$ und $R^4$ gleich oder verschieden sind und für Wasserstoff, Methyl, Ethyl, n- und i-Propyl, Fluor, Chlor, Brom, Iod, Hydroxy, Methoxy, Ethoxy, n-Propoxy, i-Propoxy, Mercapto, Methylthio, Ethylthio, n-Butylsulfinyl, Methylsulfonyl, Nitro, unsubstituiertes oder einfach oder zweifach, gleich oder verschieden durch Fluor, Chlor, Brom, Methyl, Ethyl, i-Propyl oder Trifluormethyl substituiertes Phenyl, ferner für Amino, Methylamino, Ethylamino, 2,6-Dichlorbenzylamino, Dimethylamino, Diethylamino, unsubstituiertes oder einfach oder zweifach, gleich oder verschieden durch Fluor, Chlor, Brom, Methyl, Ethyl, i-Propyl oder Trifluormethyl substituiertes Phenylamino oder für die Gruppierungen -NH-CO-$R^7$, -NH-CY'-X'-$R^8$, -NH-$SO_2R^9$, -$Y^1$-CO-$R^{10}$ -$Y^2$-CO-$X^1$-$R^{11}$, -$Y^3$-CS-$X^2$-$R^{12}$ oder -CO-$R^{13}$ stehen,
worin
$R^7$, $R^8$, $R^9$, $R^{10}$, $R^{11}$, $R^{12}$ und $R^{13}$ gleich oder verschieden sind und für Methyl, Ethyl, n- und i-Propyl, n-, i-, s- und t-Butyl oder für gegebenenfalls einfach oder zweifach, gleich oder verschieden durch Fluor, Chlor, Brom, Nitro, Methyl, Ethyl substituiertes Phenyl stehen,
Y, $Y^1$, $Y^2$ und $Y^3$ gleich oder verschieden sind und für Sauerstoff oder Schwefel stehen und
X', $X^1$ und $X^2$ gleich oder verschieden sind und für Sauerstoff, Schwefel oder für die Gruppe N-$R^{14}$ stehen,
worin
$R^{14}$ für Wasserstoff oder Methyl steht und
$R^6$ für unsubstituiertes oder einfach bis dreifach, gleich oder verschieden durch Fluor, Chlor, Brom, Methyl, Ethyl, Methoxy, Ethoxy oder Trifluormethyl substituiertes Phenyl steht.

5. Verfahren zur Herstellung von 2-Aryl-substituierten 1,10-Phenanthrolinen der Formel (Ia)

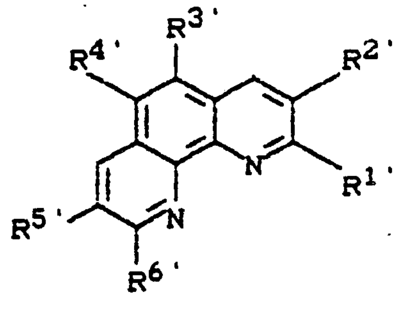

(Ia)

in welcher
$R^1$ für Wasserstoff, Alkyl oder unsubstituiertes oder substituiertes Phenyl steht,

$R^{2'}$ und $R^{5'}$ gleich oder verschieden sind und für Wasserstoff oder Alkyl stehen,

$R^{3}$ und $R^{4}$ gleich oder verschieden sind und für Wasserstoff, Alkyl, Halogenalkyl, Halogen, Hydroxy, Alkoxy, Mercapto, Alkylthio, Alkylsulfinyl, Alkylsulfonyl, Nitro, unsubstituiertes oder substituiertes Aryl, Amino, Alkylamino, unsubstituiertes oder substituiertes Arylamino, Aralkylamino, Dialkylamino, Diaralkylamino oder für die Gruppierungen $-NH-CO-R^{7}$, $-NH-CY-X-R^{8}$, $-NH-SO_2-R^{9}$, $-Y^{1}-CO-R^{10}$, $-Y^{2}-CO-X^{1}-R^{11}$, $-Y^{3}-CS-X^{2}-R^{12}$ oder $-CO-R^{13}$ stehen,

worin

$R^{7'}$, $R^{8'}$, $R^{9'}$, $R^{10'}$, $R^{11'}$, $R^{12'}$ und $R^{13'}$ gleich oder verschieden sind und für Alkyl oder unsubstituiertes oder substituiertes Phenyl stehen,

Y, $Y^{1}$, $Y^{2}$ und $Y^{3}$ gleich oder verschieden sind und für Sauerstoff oder Schwefel stehen und

X, $X^{1}$ und $X^{2}$ gleich oder verschieden sind und für Sauerstoff, Schwefel oder für die Gruppe $N-R^{14'}$ stehen, worin $R^{14}$ für Wasserstoff oder Alkyl steht,

und

$R^{6}$ für unsubstituiertes oder substituiertes Phenyl steht, sowie deren Säureadditionssalze und Metallsalzkomplexe,

ausgenommen die Verbindungen 2,9-Bis-(4-methoxyphenyl)-1,10-phenanthrolin, 2,9-Diphenyl-1,10-phenanthrolin, 2,9-Bis-(p-hydroxyphenyl)-1,10-phenanthrolin, 2,9-Bis-[4-(2-propinyloxy)-phenyl]-1,10-phenanthrolin und 2-Phenyl-1,10-phenanthrolin, dadurch gekennzeichnet, daß man

A) 8-Aminochinoline der Formel (II)

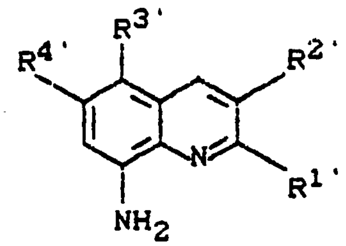

(II)

in welcher

$R^{1'}$, $R^{2'}$, $R^{3}$ und $R^{4}$ die oben angegebene Bedeutung haben,

mit α,ß-ungesättigten Aldehyden der Formel (III),

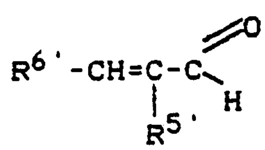

(III)

in welcher

$R^{5}$ und $R^{6}$ die oben angegebene Bedeutung haben,

gegebenenfalls in Gegenwart von Verdünnungsmitteln sowie gegebenenfalls in Gegenwart einer starken Säure oder gegebenenfalls in Gegenwart eines Oxidationsmittels oder unter Wasserabscheidung gegebenenfalls in Gegenwart eines Katalysators cyclisiert und die so erhaltenen Produkte gegebenenfalls in Säureadditions-Salze und Metallsalz-Komplexe überführt,

oder daß man

B) substituierte 1,10-Phenanthrolin-Derivate der Formel (Ib),

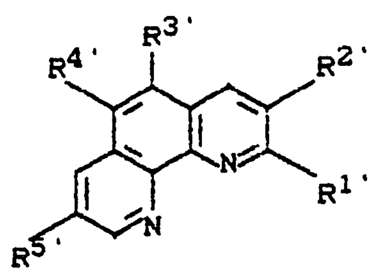

(Ib)

in welcher , , ,
$R^1$ ,$R^2$ , $R^3$ , $R^4$ und $R^5$ die oben angegebene Bedeutung haben,
in allgemein üblicher Art und Weise
α) mit Lithiumverbindungen der Formel (IV)
$R^6$ - Li (IV)
in welcher
$R^6$ die oben angegebene Bedeutung hat,
oder
β) mit Magnesium-organischen Verbindungen der Formel (IVa)
$R^6$ - Mg - X (IVa)
in welcher
$R^6$ die oben angegebene Bedeutung hat und
X für Chlor, Brom oder Iod steht,
gegebenenfalls in Gegenwart von Verdünnungsmitteln und gegebenenfalls in Gegenwart eines Inertgases umsetzt, anschließend hydrolysiert, danach mit einem Oxidationsmittel dehydriert und die so erhaltenen Produkte gegebenenfalls in deren Säureadditions-Salze oder Metallsalz-Komplexe überführt.

6. Schädlingsbekämpfungsmittel, gekennzeichnet durch einen Gehalt an mindestens einem 2-Aryl-substituierten 1,10-Phenanthrolin der Formel (I) oder (Ia) nach den Ansprüchen 1 bis 5.

7. Verfahren zur Bekämpfung von Schädlingen, dadurch gekennzeichnet, daß man 2-Aryl-substituierte 1,10-Phenanthroline der Formel (I) oder (Ia) nach den Ansprüchen 1 bis 5 auf Schädlinge und/oder ihren Lebensraum einwirken läßt.

8. Verfahren zur Herstellung von Schädlingsbekämpfungsmitteln, dadurch gekennzeichnet, daß man 2-Aryl-substituierte 1,10-Phenanthroline der Formel (I) oder (Ia) nach den Ansprüchen 1 bis 5 mit Streckmitteln und/oder oberflächenaktiven Mitteln vermischt.

Europäisches Patentamt

# EUROPÄISCHER RECHERCHENBERICHT

Nummer der Anmeldung

EP 89 11 6497

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | TETRAHEDRON LETTERS, Band 28, Nr 47, 1987, Seiten 5829-5832, Pergamon Journals Ltd, Oxford, GB. [S. NOBLAT et al] "Synthesis of an oblique bis-porphyrin system containing a 1,10 phenanthroline spacer" * Seite 5831, Verbindungen 4-6 * | 2-5 | C 07 D 471/04<br>A 01 N 43/90 //<br>(C 07 D 471/04<br>C 07 D 221:00<br>C 07 D 221:00 ) |
| X | CHEMICAL ABSTRACTS, Band 80, Nr. 17, 29. April 1974, Seite 412, Zusammenfassung Nr. 95913p, Columbus, Ohio, US; & CS-A-150 747 (B. ZAK) 15-10-1973 (Kat. D,X) | 2-4 | |
| P,A | EP-A-0 282 893 (BAYER AG) * Seite 3, Zeilen 0-49, Ansprüche 1-6,10 * | 1,5-8 | |
| | | | **RECHERCHIERTE SACHGEBIETE (Int. Cl.5)** |
| | | | C 07 D 471/00<br>A 01 N |

Der vorliegende Recherchenbericht wurde für alle Patentansprüche erstellt

| Recherchenort | Abschlußdatum der Recherche | Prüfer |
|---|---|---|
| DEN HAAG | 03-01-1990 | VOYIAZOGLOU D. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument